# EUROPEAN PATENT SPECIFICATION

(11) **EP 2 416 698 B1**
(45) Date of publication and mention of the grant of the patent: **04.09.2013**
(21) Application number: 10714695.3
(22) Date of filing: 02.04.2010
(51) Int. Cl.: A61B 5/00, A61B 5/0484, A61B 5/16

(54) **METHOD AND SYSTEM FOR TRAINING OF PERCEPTUAL SKILLS USING NEUROFEEDBACK**
VERFAHREN UND SYSTEM ZUR BEHANDLUNG VON WAHRNEHMUNGSFÄHIGKEITEN MITTELS NEUROFEEDBACK
PROCÉDÉ ET SYSTÈME D'ENTRAÎNEMENT DE FACULTÉS SENSORIELLES UTILISANT UNE RÉTROACTION NEURONALE

(30) Priority: 06.04.2009 NL 2002717
(43) Date of publication of application: 15.02.2012
(73) Proprietor: Stichting Katholieke Universiteit, Radboud Universiteit Nijmegen, 6525 HP Nijmegen (NL)
(72) Inventor: DESAIN, Petrus Wilhelmus Maria, NL-6523 HW Nijmegen (NL); MCQUEEN, James Maurice, NL-6525 HR Nijmegen (NL)
(74) Representative: Nederlandsch Octrooibureau
(86) International application number: PCT/NL2010/050171
(87) International publication number: WO 2010/117264

(56) References cited:
- WO-A1-2008/065239
- US-A- 4 216 781
- US-A- 5 724 987
- US-A1- 2003 004 429

## Description

### Field of the invention

The present invention relates to a method for training of a perceptual skill, comprising measuring electrophysiological activity in reaction to a sequence of perceptual stimuli. An electrophysiological activity signal that can be measured is e.g. an electro-encephalogram signal (EEG) measured using EEG techniques which are known as such. More in particular, the present invention relates to a method as defined in the preamble of claim 1.

### Prior art

American patent publication US2005/0085744 discloses the use of P300 signal elements for a man-machine-interface which may be used in the area of rehabilitation. Audio-visual stimuli are applied to a subject to be examined, and event related potential sare measured using EEG techniques. In some embodiments, odd ball series of stimuli are used, and the presence or absence of an expected P300 signal in a measured EEG signal is determined (which allows control of e.g. equipment).

American patent publication US2003/004429 discloses a process for modifying the features of electrophysiological activity in response to a stimulus, such as a sequence of tones (auditory oddball paradigm).

American patent US 5,724,987 discloses a computer based training system using EEG measurements. The results of the measurements are used to modify a training protocol applied to a subject. EEG signals are being used to measure attention and cognitive load.

American patent US6,795,724 discloses a neurofeedback system, in which characteristics of measured EEG data such as frequency or amplitude are processed and presented to the test person as a color on a screen.

American patent application US2004/0152995 discloses a method and system for diagnostic and therapeutic use of neurofeedback for treating disorders associated with impaired attention, like ADHD. Physiological EEG data acquisition is used and a sequential stochastic model procedure in order to assess EEG inconsistencies.

US2004/131998 (Marom e.a.) discloses a method for training a biological neural network, such as the human brain. The neural network is being trained until a desired output is obtained, after which a resting period is applied. Applications disclosed include learning to control protheses for humans.

W02006/021952 (Re-ability, Inc.) discloses a rehabilitation device and a training method for motor training of test persons. EEG-measurements are used to provide feedback to a test person.

### Summary of the invention

The present invention seeks to provide an improved training method and system for perceptual skills, such as auditory skills, using neurofeedback during the training.

According to the present invention, a method according to claim 1 is provided. The method comprises matching the measured electrophysiological activity signal with a predefined electrophysiological signature signal, in which the predefined electrophysiological signature signal corresponds to an early electrophysiological component, and providing (instantaneous) feedback when a match is detected. With this method embodiment, a brain computer interface (BCI) is used to detect an early response, and the perceptual skill is e.g. an auditory skill.

Different forms of neurofeedback already have a place in the treatment of epilepsy and ADHD, and have been used to improve the performance of musicians and athletes. The present application extends the application of neurofeedback systems to a new set of educational possibilities. It aims at tasks related to perceptual skills that are hard and expensive to train, such as learning new auditory patterns in language and music. The market of foreign language learners and music students is large. A method and system which can effectively improve the perception of subtle categorical differences in musical and linguistic domains would also allow application of the present method and system to other tasks, including training of the analysis of medical and other complex visual images, as well as the monitoring of complex systems. Attaining a high level of expertise in these domains requires years of training. The successful application of neurofeedback to this training would not only expedite the process but also add an individualized component, as not all users will experience the same sets of difficulties. The present method and system will in fact directly adapt to individual users based on their current brain activity. Different types of higher-education facilities would provide a large base for the potential deployment of BCI-based training system (Brain Computer Interface). The present method and system embodiments, which target the healthy population and seeks to solve everyday learning problems, will considerably broaden the scope of BCI.

The BCI based method and system use neurofeedback, including the measurement of electrophysiological activity on a (human) test subject. Such electrophysiological activity may be measured using known EEG measurement techniques. As compared to expensive fMRI or MEG devices, an EEG device is more affordable and accessible (e.g. for schools and learning institutes). Furthermore, EEG devices are quickly being equipped with various advanced functions, such as stable active electrodes, fast fit caps, and compact mobile setups.

In an embodiment, the measured electrophysiological activity signal is an electro-encephalogram (EEG) signal. E.g. the early electrophysiological component comprises an EEG signal related to perception, such as the mismatch negativity signal (MMN). As an alternative, the early electrophysiological component comprises an EEG signal related to attention, such as the P300 signal. The method may additionally or alternatively further use EEG signal analysis, such as independent component analysis (ICA), wavelet de-noising, or pre-processing using spatial filtering, beam forming, automatic artifact rejection.

In a further embodiment, multiple sequences of stimuli are used to determine the predefined electrophysiological signature signal from a number of electrophysiological activity measurements.

In further embodiments, the feedback is dependent on the strength of the measured electrophysiological signal, e.g. blurriness of visual feedback image may be introduced dependent on the measurement outcome. The method may be used to start with known EEG components, and detect these in the measured signals. Using knowledge before executing the experiment (e.g. it is known which EEG components should occur after a certain stimulus), the strength of the actually measured signals is used as feedback. As an alternative, two classes of stimuli are used, for which the difference in response can be calculated and provided as feedback.

In an even further embodiment, the sequence of perceptual stimuli comprises a number of stimuli of a first category and a stimulus of a second type occasionally appearing in the sequence of stimuli (a so called oddball sequence).

The method in a further embodiment further comprises generating a new sequence of perceptual stimuli, in which the difference between stimuli of the first type and the stimulus of the second type is dependent on the strength of the measured electrophysio logical signal in the previous sequence. By adapting the difference between stimuli in a sequence it is possible to train efficiently based on detectability of the responses.

The perceptual stimuli may in various embodiments relate to one of the group of: a category of pitch patterns, a category of timing patterns, a category of music patterns (pitch, rhythm). In general any distinctive auditory characteristic may be used like pitch, timing, timbre, or even using other sensory channels (vision, tactile,...) This may be very helpful when learning new auditory patterns in a language and/or music, e.g. lexical tone in Chinese language and mora timing in Japanese language.

In a further aspect, the present invention relates to a brain computer interface learning device comprising a stimuli generator for providing perceptual stimuli to a test subject, a sensor assembly for measuring electrophysiological activity on the test subject, a processing unit connected to the sensor assembly, and a feedback unit connected to the processing unit for providing perceptual feedback to the test subject, in which the processing unit is arranged to control the stimuli generator, sensor assembly and feedback unit for executing the method according to any one of the present method embodiments.

Applications related to the use of neurofeedback for training perceptual skills, as described in the present invention embodiment, or systems which made use of the oddball-paradigm in conjunction with online EEG-based feedback. No prior art system has made the specific step of using online neurofeedback based on components of the EEG signal related to an early electrophysiological component associated with the sequence of perceptual stimuli, such as perception (MMN) and attention (P300), for use in the training of perceptual skills, e.g. related to music and language.

### Short description of drawings

The present invention will be discussed in more detail below, using a number of exemplary embodiments, with reference to the attached drawings, in which
Fig. 1 shows a block diagram of a neurofeedback system in which the present invention may be embodied;
Fig. 2 shows a block diagram of an embodiment of the brain computer interface (BCI) learning device according to an embodiment of the present invention.

### Detailed description of exemplary embodiments

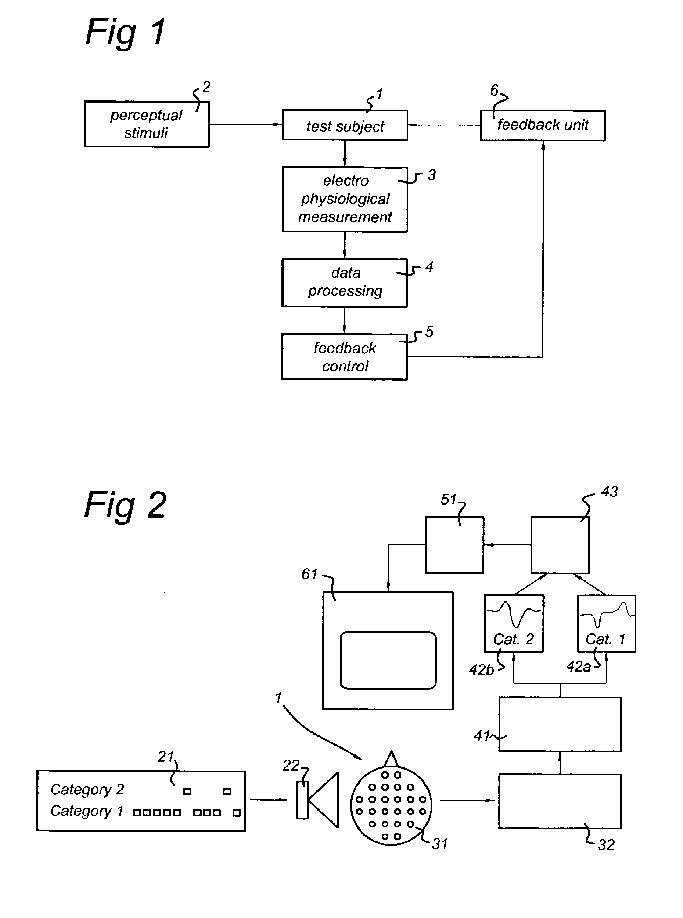

In Fig. 1 a schematic block diagram is shown of a neurofeedback system as used to implement the present invention embodiments. A test subject 1 is subjected to a sequence of perceptual stimuli provided by a stimuli generator 2. The neurological response of the test subject 1 (e.g. in the form of an electrophysio logical activity signal) is measured using a sensor assembly 3, e.g. using electro-encephalogram (EEG) measurements. The data obtained by the sensor assembly 3 is processed in a data processing unit 4, and appropriate result data is output to a feedback control block 5. The feedback control block 5 (which could also be regarded as part of the data processing unit 4) is connected to and controls a feedback unit 6, which provides a feedback to the test subject 1, e.g. in the form of a visual presentation or an auditory signal.

Learning to perceive the sounds of a foreign language requires many hours of training, and non-native adult listeners rarely achieve native-like levels of performance. This may be because of inadequate feedback during training. In the present invention embodiments, a solution for this problem is provided by developing a new device that provides trainees (test subject 1) with feedback based on the activity of their brains. In existing training protocols, feedback is limited because it is based on the listener's overt behaviour (e.g., whether a categorization decision was correct). This means that there is a delay between stimulus processing and presentation of feedback and that feedback is provided only on explicit responses. This is not optimal: It would be better if the feedback loop was faster and more direct. Developments in Brain Computer Interfacing (BCI) techniques now make this possible. In the exemplary embodiments described below, an innovative BCI device is presented that assists in the learning of novel auditory categories in speech and in music.

Electrophysiological activity corresponding to early perceptual analysis will be measured (using sensor assembly 3, Fig. 1) and analyzed in real time (data processing unit 4), and reinforcing visual feedback based on that activity will be given to listeners 1 with no delay (feedback unit 6). The immediate feedback about the brain activity is expected to reinforce the categorization behaviour. Experiments will train perception of sounds: Experiments on speech will examine learning about pitch (e.g. lexical tone in Mandarin Chinese) and rhythm (e.g. mora timing in Japanese). Parallel experiments on music will also examine learning about pitch (the Gamelan system) and rhythm (Jazz swing). In the embodiments as discussed in more detail below robust methods for extracting, in real time, early electrophysiological components, and suitable feedback techniques are presented.

The embodiments as described below are discussed with respect to specific applications. However, a more general-purpose device is envisaged. The device is not only applicable for speech learning but also for learning other auditory or other perceptual domains. A general-purpose BCI device for perceptual skill training could thus have considerable impact on mainstream second-language and music education. Despite many efforts in the design of training methods, new perceptual categories can still not be acquired rapidly. For example, it remains hard for listeners to learn new categories of pitch patterns and timing patterns in foreign-language and in music. In the present invention embodiment, a method is provided to train perceptual processing directly, by means of a Brain Computer Interface (BCI) which is expected to speed up learning of perceptual skills in both speech and music.

There are two, inter-related reasons why learning to perceive new auditory categories may be so hard. The first is that the new categories have to be learnt in the context of existing knowledge. In the case of speech perception, the acoustical characteristics of the listener's first language are stable and highly learned. The listener tries to interpret novel stimuli as exemplary of established pitch patterns or rhythm patterns. A related problem arises in the perception of music. It is hard to perceive a new pitch system and rhythmic categories because they are often integrated into one's established pitch and rhythm systems, In both music and speech, therefore, a new acoustic contrast is hard to perceive because the new sound can readily be assimilated into an existing category. This interference with old categories is also one of the reasons why learning the sounds of a second language or of a different musical system is so much more difficult as an adult than learning these sound categories in infancy. Unlike adults, infants do not have an existing system that interferes with category acquisition.

The second reason why auditory category learning is hard is that feedback during training is impoverished. Feedback in a standard behavioural training paradigm is limited to binary information on the correctness of behavioural responses. Furthermore, those responses are based on explicit perceptual decisions. If those decisions are strongly influenced by existing category knowledge, then feedback based on those decisions alone will be a poor reinforcer. For example, if a Dutch listener tends to hear a Japanese long syllable [chiizu] (cheese) as short [chizu] (map), then, in a two-alternative forced-choice task, feedback to this listener will indicate that his/her responses are errors, but can do no more than this. The feedback does not distinguish for example between trials where the input [chiizu] was perceived to be very similar to [chizu] and trials where some difference was actually perceived. Feedback based on overt behaviour is weak with respect to information contained therein both because it is binary (correct/incorrect) and because the underlying behaviour is based on conscious perception that has been filtered by prior category knowledge. This type of feedback is also impoverished because it is delayed in time: It occurs after an overt response, long after the perceptual processing of the auditory stimulus. Thus, this type of feedback may be far from optimal in shaping crucial early perceptual processes.

According to the present invention embodiments, the solution to the problem of auditory category learning is to detect mental activity at an early stage directly from the brain, and not to wait for behavioural responses. It has been shown that the traces of perceptual processes can be measured using EEG. For instance, if, in a so-called oddball paradigm, a stimulus sound of a different category occasionally appears in a train of reference sounds, electrophysiological responses to those odd-one-out sounds can be detected. The nature of those brain responses is determined by the type of cognitive processing involved. For example, the Mismatch negativity (see below for more details) is known to stem from early perceptual processing, while the P300 (see also below for more details) responds to higher, more syntax-like, levels of regularity and violations of expectation. These classical EEG components have largely been studied using ERPs (Event Related Potentials, i.e., averaged EEG measurements across trials). However, with the advent of BCIs and more powerful signal processing methods (e.g., localization, filtering and beam forming methods), an advance towards the detection of these signatures in a small number of trials, and even in a single trial has been made. These advances make it possible to present feedback directly to the learner about the neural processes that arise in response to the odd-one-out. Via this feedback neural changes may take place, directly stimulated by the reward of developing a new category. In this way pure and direct training of perception comes within reach.

EEG components which reflect early perceptual processing should at least in part be pre-categorical, and thus are not influenced by existing categorical knowledge. Furthermore, these measurements make it possible to provide continuously graded feedback reflecting the strength of the electrophysiological response, i.e. the feedback is dependent on the strength of the measured electrophysiological signal. Feedback will be provided in the form of visual reinforcement in one of the present invention embodiments. Large perceived differences across new categories will be encouraged, while perceived differences within new categories will not. Using such signals should provide much stronger reinforcement for perceptual learning than all-or-none feedback based on a behavioural response.

In a further embodiment, another approach is to use the strength of EEG signals to control the size of difference in the present stimuli to be optimally adapted to the current state of the perceptual thresholds. E.g. for a sequence of perceptual stimuli comprising a number of stimuli of a first category and a stimulus of a second type which appears occasionally in the sequence of stimuli (odd-one-out sequence), the difference between the stimuli of the first and the second type may be made dependent on the strength of the measured electrophysiological signal. As the training progresses, the perceptual thresholds become smaller and consequently one learns to distinguish smaller differences in auditory information. This requires non-active participation of the learners, as early perceptual processing of auditory information is pre-attentive. Such a method has the potential to provide effortless training of new categories (e.g., learning while reading a book). In the current project, this passive training method will be compared with active training in a task requiring overt responses.

In Fig.2, a more detailed block diagram is shown of an embodiment of the present invention, wherein the early perceptual processing detection is applied. The blocks 1-6 as shown in Fig. 1 correspond in general to one or more of the blocks as shown in Fig. 2. In this experimental set-up, the perceptual stimuli are provided by block 21 as auditory stimuli using an odd-ball sequence as discussed above. As indicated, five different auditory stimuli of Category 1 are followed by one auditory stimulus of Category 2, then again three stimuli of Category 1, one of Category 2 and another one of Category 1. These stimuli are input to a loud speaker 22 and played to a test subject 1 (a human).

EEG electrodes 31 on the test subject 1 provide EEG data in response to the train of stimuli. In block 32, the EEG data are pre-processed, e.g. using artefact rejection, beam forming or other known EEG data processing techniques.

The pre-processed EEG data is input to a feature extractor block 41 for extracting EEG signatures. These EEG signatures are then input to two correlator blocks 42a and 42b, which are tuned to a predefined electrophysiological signature signal. The predefined electrophysiological signature signal corresponds to an early electrophysiological component associated with a response to an auditory stimulus of category 1 and category 2, respectively. The obtained results are further processed in another processing block 43, which calculates how big a difference exists between the outputs of correlator blocks 42a and 42b. This measure is then input to a feedback control block 51, in which a history window is used to provide an appropriate visual feedback signal to the test subject 1 using a visual feedback display 61.

The present invention will now also be explained in further detail using a number of practical implementations. In adult second-language learning, the discrimination of pitch patterns or timing patterns that are not native requires considerable training. For example, learning pitch-based tonal distinctions is crucial for learning Mandarin Chinese as these distinctions can determine the meanings of words (e.g., "ma" can mean four different things, depending on the syllable's pitch contour). However, learning about tone is particularly challenging for native speakers of non-tonal languages because lexical tones do not have a direct correlate in non-tonal languages. Similarly, being able to distinguish the duration of vowels and consonants is extremely important in learning Japanese as many homonyms are differentiated only by durational distinction (e.g., vowels can be short, with one mora, or long, with two morae; the mora is a timing or rhythmic unit in Japanese. This again is hard to learn for native speakers of languages that do not share such characteristics. Several training methods have been proposed and shown to be successful, but are usually very time-intensive (approximately 20 hours of training). Using the present invention embodiments, it is possible to substantially reduce the amount of time required to learn new distinction of pitch change and durational change in speech.

E.g., it is possible to focus on Dutch listeners learning prosodic distinctions based on pitch and timing information. Learning new pitch patterns and timing patterns in Dutch speakers is hard because such information is not encoded in the Dutch lexicon. The first contrast will be the distinction between the high rising pitch contour ("Tone 2") and the low dipping pitch contour ("Tone 3") of a Mandarin Chinese syllable chi [ 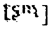 . These tones were chosen (from among the set of high level, high rising, low dipping and high falling contours) because they appear to be the most difficult for non-native listeners to discriminate and thus are the most likely to show the benefits of BCI-assisted learning. The second contrast will be based on the timing of the second vowel in the Japanese words [chizu] (where [i] is one mora) and [chiizu] (where [ii] is two morae).

In other applications, musical perception is trained, relating to pitch and timing. While in western tonal music, the smallest interval is a minor second, intervals smaller or larger than this interval are common in non-western musical cultures. Nevertheless, in both cases, the half step functions as the reference category. If a musical culture makes use of a different tuning system, different reference categories need to be learned and existing Western categories need to be abandoned. For example, the Pelog scale uses a fourth that has a different tuning from the western fourth. Western listeners have difficulties detecting deviations from this "Pelog fourth", because they have not internalized this category. Likewise, specific temporal ratios are often timed in different characteristic ways in different musical genres. A specific way of timing a rhythmic ratio becomes the expected (categorical) timing. For example, subtleties in swing ratio timing are crucial for good jazz performance. Non-trained listeners have difficulties distinguishing these differences because long-short rhythms tend to be assimilated to more prominent rhythmic categories.

An important feature of the present invention embodiments relates to the electrophysiological signature signals to be recognized as markers of mental processes. It is crucial for the success of the BCI training paradigm that reliable markers (features in the EEG signal) are extracted that signal the relevant stimulus discriminations (e.g., levels at which stimuli are classified as same or different). Furthermore, reinforcement will be most effective if it can be given on the basis of a small number of trials, and even more so if it can be given based on a single trial. Several markers have been identified in the literature and validated in offline analyses. However, their use on a single trial or on only a few trials requires sophisticated signal-(pre)processing techniques (e.g., spatial filtering, beam forming), as well as careful automatic artefact rejection.

In one specific embodiment of the present invention, multiple sequences of stimuli are used to determine the predefined electrophysiological signature signal from a number of electrophysiological activity measurements. This allows to obtain better quality electrophysiological signature signals (markers) to be used in the actual learning process.

One specific embodiment of the present invention uses an EEG signal related to perception, i.e. a marker called Mismatch Negativity (MMN). The human auditory system can pick up occasional changes (deviants) in acoustic regularities. The detection of change is reflected by an elicitation of the mismatch negativity component (MMN) measurable with EEG. When deviants are detected, a negative component of ERP is observed 150 to 250 ms after the deviant sound presentation. The amplitude of the MMN is known to be larger when detection is easier. It is known that MMN is elicited irrespective of the attentional focus of subjects. This means that the detection of deviants occurs at an early stage of sensory processing. For example, the auditory system may store the regular features of the acoustic environment as sensory memory traces and automatically compare new events with stored information. MMNs seem to be influenced by early exposure to the individual's sound environment. For example, MMNs can reflect experience with the listener's native language. Using MMN, recent studies have demonstrated that musicians are superior to non-musicians in pre-attentively extracting information about pitch tuning and rhythmic structure.

Interestingly, higher processes or cognitive mechanisms may not always detect the signal of stimulus change generated by the auditory cortex, as indicated by the observation that an MMN often precedes the behavioural discrimination itself in the course of the training. The MMN thus provides insight into the interaction between the conscious process of discrimination and the unconscious process of extracting differences. This nature of the MMN makes it particularly interesting for the present invention embodiments.

A further specific embodiment of the present invention uses an EEG signal related to attention, i.e. the well known P300 EEG signal component. The P300 is a class of components that are observed in response to expectancy violations (see e.g. US patent US5,137,027). This response occurs when the stimuli are attended to in violation-detection tasks. The latency and the size of the response are affected by factors such as task difficulty, stimulus expectancy, and stimuli content. The P300 may occur in response to deviations in perceptual qualities as well as to violations of higher-order expectancies concerning for example the syntax of language and music. In these latter cases, the positive component is regularly observed to occur as late as around 600 ms. This P600 is sometimes treated as a separate class related to working memory effort to integrate the deviant into the context.

Characteristic of the P300 is that the amplitude and latency of the positive component varies with attention, task demands and stimulus expectancy. This enables us to use this component as a measure of the subjective degree of expectancy violation. Training may increase the P300 response to violations, as suggested by previously observed systematic differences between brain responses of musically trained and non-trained participants.

Existing BCIs which have achieved online success on a single trial level have done so predominantly using imagined movements as a task in combination with algorithms for processing the EEG signal (see e.g. US patent US7,120,486). On the other hand, BCIs which make use of event-related potentials such as the P300 have also seen a recent reduction in the number of trials required for successful identification of the target signal. The initial system by Farwell and Donchin tested several signal processing algorithms, and was able to achieve high classification rates of the P300 signal in as few as five trials. Recently, improvements to the P300 speller systems have been made by applying support vector machine techniques (SVM), and have reduced the number of trials required for reliable classification to between two and three.

Moreover, recent approaches to the analysis of ERPs using independent component analysis and wavelet de-noising have made significant advances in reducing the problems caused by the variability of the ERP across trials for data analysis purposes. This made it possible to conduct an offline analysis of ERP data from an oddball task possible at the single-trial level, specifically for the purposes of tracking learning.

However, in order to ensure that the development of the ERP-based neurofeedback paradigm proceeds, initial offline analyses of pilot data from the proposed experimental tasks will be used in order to assess the success of both multiple-trial and single-trial techniques for calculating ERPs. Based on the results of these analyses, the techniques demonstrating the most reliable classification of the ERP signals will then be implemented in an embodiment of the present invention. Although the use of a reliable single-trial classifier is obviously better, a reliable classifier which requires a small number of trials, as in existing ERP-based BCIs, would also be able to generate feedback on low-level, pre-categorical perceptual processes, and is still hypothesized to provide a significant increase in the rate of learning of new auditory categories when compared to traditional behavioural feedback.

A further feature of the present invention embodiments relates to the feedback provided to the test subject 1 (blocks 51 and 61 in Fig. 2). In traditional neurofeedback research, it is typical for the feedback to come in the form of a sound or a visual image. The specific type of sound or image used in different applications may vary, but the basic principle remains the same: It is based on operant conditioning, a technique that has been used widely to modify behaviour. The presentation of the sound or image upon a detected increase of the target frequency band serves as a reward, and reinforces the mental activity of the user which occurred immediately beforehand.

In the present invention embodiment, existing neurofeedback protocols are extended by generating neurofeedback based on the MMN and P300 ERPs. Simple visual feedback (using display 61) is presented as a means of reinforcement when test subjects 1 successfully detect deviant target stimuli 21 as indicated by an MMN or P300 component in the ERP data. Previous research on brain-computer interfaces has shown that the P300 ERP component can be used as a control signal for a spelling device using a small number of trials to acquire the data needed for the calculation of the ERP. In BCI research the output of a system is often a discrete symbolic classification. This would only be useful as a success-fail feedback signal. However, most approaches also deliver a confidence measure of the inferred class, or a probability that the inferred class was indeed present as the stimulus. These measures can be used to provide continuous feedback which will be a stronger reward signal to pick up on yet-unlearned and subtle category distinctions. The continuous feedback could take the form of e.g., blurriness of a visual image on the feedback display 61.

Several features are relevant in one or more of the present invention embodiment. First, a technique is provided that can reliably extract the relevant EEG signatures, on the basis of just a few perception trials. Stimuli will be presented in pilot training experiments without neurofeedback, but with EEG measurement. Listeners will be presented with a sequence of speech sounds (or musical sounds) of the same type, with occasional odd-one-out stimuli from the other category. Signal processing techniques will be applied to these EEG data to isolate candidate mismatch-detection signatures. These pilots will also provide baseline measures of learnability without a BCI, and thus allow for further refinement of stimulus materials. The BCI system is also able to map the extracted EEG signatures to appropriate visual features for feedback.

## Claims

1. Method for training of a perceptual skill, comprising
- measuring electrophysiological activity in reaction to a sequence of perceptual stimuli,
- matching the measured electrophysiological activity signal with a predefined electrophysiological signature signal, in which the predefined electrophysiological signature signal corresponds to an early electrophysiological component, and
- providing feedback when a match is detected, wherein
- the sequence of perceptual stimuli comprises a number of stimuli of a first category and a stimulus of a second type occasionally appearing in the sequence of stimuli, and **characterized in that** it
- further comprises generating a new sequence of perceptual stimuli, in which the difference between stimuli of the first type and the stimulus of the second type is dependent on the strength of the measured electrophysiological signal.

2. Method according to claim 1, in which the measured electrophysiological activity signal is an electro-encephalogram (EEG) signal.

3. Method according to claim 1 or 2, in which the early electrophysiological component comprises an EEG signal related to perception.

4. Method according to claim 1 or 2, in which the early electrophysiological component comprises an EEG signal related to attention.

5. Method according to any one of claims 1-4, in which multiple sequences of stimuli are used to determine the predefined electrophysiological signature signal from a number of electrophysiological activity measwements.

6. Method according to any one of claims 1-5, in which the feedback is dependent on the strength of the measured electrophysiological signal.

7. Method according to any one of claims 1-6, in which the perceptual stimuli relate to one of the group of: a category of pitch patterns, a category of timing patterns, a category of music patterns.

8. Brain computer interface learning device comprising
- a stimuli generator for providing perceptual stimuli to a test subject
- a sensor assembly for measuring electrophysiological activity on the test subject
- a processing unit connected to the sensor assembly; and
- a feedback unit connected to the processing unit for providing perceptual feedback to the test subject,
**characterized in that** the processing unit is arranged to control the stimuli generator, sensor assembly and feedback unit for executing the method according to any one of claims 1-7.

## Patentansprüche

1. Verfahren zum Trainieren einer Wahrnehmungsfähigkeit, umfassend
- Messen von elektrophysiologischer Aktivität in Reaktion auf eine Abfolge von Wahrnehmungsreizen,
- Abgleichen des gemessenen elektrophysiologischen Aktivitätsignals mit einem vordefinierten elektrophysiologischen Signatursignal, wobei das vordefinierte elektrophysiologische Signatursignal einer frühen elektrophysiologischen Komponente entspricht, und
- Bereitstellen einer Rückmeldung, wenn eine Übereinstimmung nachgewiesen wird, wobei
- die Abfolge von Wahrnehmungsreizen eine Anzahl von Reizen einer ersten Katogorie und einen Reiz eines zweiten Typs, der gelegentlich in der Abfolge von Reizen erscheint, umfasst, und **dadurch gekennzeichnet, dass** es
- weiterhin Erzeugen einer neuen Abfolge von Wahrnehmungsreizen umfasst, in der der Unterschied zwischen Reizen des ersten Typs und dem Reiz des zweiten Typs von der Stärke des gemessenen elektrophysiologischen Signals abhängt.

2. Verfahren nach Anspruch 1, in dem das gemessene elektrophysiologische Aktivitätsignal ein Elektroenzephalogramm (EEG)-Signal ist.

3. Verfahren nach Anspruch 1 oder 2, in dem die frühe elektrophysiologische Komponente ein auf Wahrnehmung bezogenes EEG-Signal umfasst.

4. Verfahren nach Anspruch 1 oder 2, in dem die frühe elektrophysiologische Komponente ein auf Aufmerksamkeit bezogenes EEG-Signal umfasst.

5. Verfahren nach einem beliebigen der Ansprüche 1-4, in dem mehrere Abfolgen von Reizen verwendet werden, um das vordefinierte elektrophysiologische Signatursignal aus einer Anzahl von elektrophysiologischen Aktivitätsmessungen zu bestimmen.

6. Verfahren nach einem beliebigen der Ansprüche 1-5, in dem die Rückmeldung von der Stärke des gemessenen elektrophysiologischen Signals abhängt.

7. Verfahren nach einem beliebigen der Ansprüche 1-6, in dem die Wahrnehmungsreize auf eine der Gruppen bezogen sind: eine Kategorie von Tonhöhenmustern, eine Kategorie von zeitlichen Mustern, eine Kategorie von musikalischen Mustern.

8. Gehirn-Computer-Schnittstellen-Lerngerät, umfassend
- einen Reizerzeuger zum Bereitstellen von Wahrnehmungsreizen an ein Testsubjekt
- eine Sensoranordnung zum Messen von elektrophysiologischer Aktivität am Testsubjekt
- eine Verarbeitungseinheit, die mit der Sensoranordnung verbunden ist;
und
- eine Rückmeldungseinheit, die mit der Verarbeitungseinheit verbunden ist, um dem Testsubjekt Wahrnehmungsrückmeldung bereitzustellen, **dadurch gekennzeichnet, dass** die Verarbeitungseinheit angeordnet ist, um Reizerzeuger, Sensoranordnung und Rückmeldungseinheit zum Ausführen des Verfahrens gemäß einem beliebigen der Ansprüche 1-7 zu steuern.

## Revendications

1. Procédé d'entraînement d'aptitudes de perception, comprenant les étapes consistant à :
- mesurer une activité électrophysiologique en réaction à une séquence de stimuli de perception ;
- mettre en correspondance le signal d'activité électrophysiologique mesurée avec un signal de signature électrophysiologique prédéfinie, dans lequel le signal de signature électrophysiologique prédéfinie correspond à une composante électrophysiologique antérieure ; et
- émettre une rétroaction quand une correspondance est détectée, dans lequel
- la séquence de stimuli de perception comprend un certain nombre de stimuli d'une première catégorie et un stimulus d'un second type qui apparait ponctuellement dans la séquence de stimuli, et **caractérisé en ce que** :
- il comprend en outre une étape consistant à générer une nouvelle séquence de stimuli de perception, dans lequel la différence entre les stimuli du premier type et le stimulus du second type, dépend de la force du signal électrophysiologique mesuré.

2. Procédé selon la revendication 1, dans lequel le signal d'activité électrophysiologique mesurée est un signal d'électroencéphalogramme (EEG).

3. Procédé selon la revendication 1 ou la revendication 2, dans lequel la composante électrophysiologique antérieure comprend un signal d'EEG qui se rapporte à la perception.

4. Procédé selon la revendication 1 ou la revendication 2, dans lequel la composante électrophysiologique antérieure comprend un signal d'EEG qui se rapporte à l'attention.

5. Procédé selon l'une quelconque des revendications 1 à 4, dans lequel de multiples séquences de stimuli sont utilisées de façon à déterminer le signal de signature électrophysiologique prédéfinie à partir d'un certain nombre de mesures d'activité électrophysiologique.

6. Procédé selon l'une quelconque des revendications 1 à 5, dans lequel la rétroaction dépend de la force du signal électrophysiologique mesuré.

7. Procédé selon l'une quelconque des revendications 1 à 6, dans lequel les stimuli de perception se rapportent à l'une du groupe constitué par : une catégorie de motifs de ton, une catégorie de motifs de synchronisation, une catégorie de motifs de musique.

8. Dispositif d'apprentissage d'interface cerveau - ordinateur comprenant :
- un générateur de stimuli destiné à fournir des stimuli de perception à un sujet d'expérience ;
- un ensemble de capteur destiné à mesurer l'activité électrophysiologique chez le sujet d'expérience ;
- une unité de traitement connectée à l'ensemble de capteur ; et
- une unité de rétroaction connectée à l'unité de traitement destinée à fournir une rétroaction de perception au sujet d'expérience ;
**caractérisé en ce que** l'unité de traitement est agencée de façon à commander le générateur de stimuli, l'ensemble de capteur et l'unité de rétroaction, de façon à exécuter le procédé selon l'une quelconque des revendications 1 à 7.
